# FASCICULE DE BREVET EUROPEEN

(11) **EP 0 699 430 B1**
(45) Date de publication et mention de la délivrance du brevet: **02.10.1996**
(21) Numéro de dépôt: 95401633.3
(22) Date de dépôt: 06.07.1995
(51) Int. Cl.: A61K 7/06

(54) **Procédé de traitement des fibres kératiniques humaines à l'aide d'amides à chaîne grasse et de vapeur d'eau**
Verfahren zur Behandlung der menschlichen keratinischen Fasern mit Amiden mit Fettkette und mit Wasserdampf
Method of hair treatment using amides containing fatty chains and steam

(30) Priorité: 02.08.1994 FR 9409583
(43) Date de publication de la demande: 06.03.1996
(73) Titulaire: L'OREAL, F-75008 Paris (FR)
(72) Inventeur: Braida, Valério Damarys, F-75004 Paris (FR); Sturla, Jean-Michel, F-92210 Saint-Cloud (FR)
(74) Mandataire: Tezier Herman, Béatrice

(56) Documents cités:
- WO-A-93/02656
- FR-A- 2 273 492

## Description

La présente invention concerne un procédé amélioré de traitement des fibres kératiniques humaines, en particulier des cheveux, en vue notamment de diminuer et/ou d'empêcher les dégradations de ces dernières, ledit procédé étant notamment utilisable dans le domaine des salons de coiffure, de beauté, de cosmétique et analogues, professionnels. Plus précisément encore, elle concerne un procédé mettant en oeuvre de la vapeur d'eau et des substances traitantes particulières à base d'un composé amide possédant au moins une chaîne grasse à l'exception du diéthanolamide de coprah.

Il est bien connu que les cheveux peuvent être généralement sensibilisés (i.e. abîmés) à des degrés divers par l'action des agents atmosphériques et/ou de traitements capillaires, mécaniques ou chimiques, tels que des colorations, des décolorations et/ou des permanentes. Des cheveux ainsi sensibilisés deviennent souvent difficiles à démêler et à coiffer.

Ainsi, dans le but de traiter et/ou de protéger la fibre capillaire, on a déjà proposé d'utiliser des amides possédant une chaîne grasse (voir WO-A-9 302 656). Toutefois, la tenue de ces produits sur les cheveux peut apparaître comme encore insuffisante.

En particulier, on constate que l'utilisation d'un simple shampooing élimine une grande partie des amides qui se sont déposés sur les cheveux.
Compte tenu du fait que la protection et/ou le soin apportée par les amides est d'autant plus élevé que leurs quantités présentes sur les cheveux sont importantes, la demanderesse a donc cherché à améliorer la fixation des amides sur et/ou dans le cheveux.

Ainsi, à la suite d'importantes recherches menées sur la question, il a maintenant été trouvé par la Demanderesse, et ceci de façon tout à fait inattendue et surprenante, que ces buts, et d'autres, pouvaient être atteints en mettant en oeuvre, selon certaines conditions particulières, de la vapeur d'eau sur des fibres kératiniques humaines préalablement traitées avec des amides possédant au moins une chaîne grasse à l'exception du diéthanolamide de coprah. Cette découverte est à la base de la présente invention.

Le procédé selon l'invention permet d'augmenter la quantité d'amide fixée sur les cheveux. En effet, la quantité d'amides restant sur les cheveux après un rinçage ou un shampooing est plus importante dans le cadre du procédé selon l'invention que lorsqu'on n'utilise pas de vapeur d'eau chaude.
Ce procédé permet également d'améliorer les propriétés cosmétiques en particulier la douceur et le lissage des cheveux.

Ainsi, il est maintenant proposé selon la présente invention un nouveau procédé de traitement des fibres kératiniques humaines, et en particulier des cheveux, ledit procédé étant caractérisé par le fait qu'il consiste (i) à mettre en contact, pendant une durée n'excédant pas 2 minutes, un gaz contenant de la vapeur d'eau et dont la température est d'au moins 75°C, avec des fibres kératiniques humaines sur lesquelles on a appliqué une composition contenant au moins un composé amide possédant au moins une chaîne grasse à l'exception du diéthanolamide de coprah, (ii) à refroidir les fibres ainsi traitées.

Bien que l'exposé qui suit s'articule essentiellement autour du cas particulier du traitement des cheveux, on notera ici que le procédé selon l'invention est applicable à toute matière kératinique humaine en général, notamment cils, moustaches, poils et autres.

Le gaz chaud traitant contient de préférence au moins 1% en volume de vapeur d'eau.
En plus de la vapeur d'eau, le gaz vecteur (ou support) peut contenir de la vapeur de solvant, ainsi que des gaz tels que l'oxygène ou l'azote, des mélanges de gaz tels que l'air ou bien encore d'autres composés vaporisables.

A titre de solvants qui peuvent être avantageusement utilisés pour la production de vapeur (mélanges eau-solvant), on fait plus particulièrement appel aux solvants organiques cosmétiquement acceptables, comme par exemple des alcools tels que l'éthanol, l'isopropanol, l'alcool benzylique, l'alcool phényléthylique ou des glycols ou éthers de glycol tels que par exemple l'éthylène glycol et ses éthers monométhylique, monoéthylique et monobutylique, le propylène glycol, le butylène glycol, le dipropylèneglycol, ainsi que les alkyléthers comme le monobutyléther du diéthylèneglycol.

Selon la présente invention, le gaz est constitué de préférence soit uniquement ou essentiellement de vapeur d'eau, soit d'un mélange d'eau et d'air.

La température du gaz est de préférence supérieure ou égale à 85°C, et est plus particulièrement comprise entre 85°C et 150°C environ.

Selon une caractéristique importante du procédé selon l'invention, le temps de contact entre le gaz chaud traitant et la fibre doit être bref, et en tout cas ne pas excéder 2 minutes. De préférence, le gaz est mis en contact avec la fibre pendant une durée allant de 0,01 seconde à 30 secondes, et encore plus préférentiellement de 1 seconde à 10 secondes. Bien entendu, l'application du gaz peut être répétée plusieurs fois sur la même fibre, chaque opération se faisant selon une durée telle qu'indiquée ci-dessus.

Un mode préféré de réalisation du procédé selon l'invention consiste à appliquer tout d'abord sur les cheveux une composition contenant des amides à chaîne grasse, puis à soumettre ces mèches ainsi imprégnées de céramides à l'action brève de la vapeur d'eau selon les conditions mentionnées ci-avant, puis à refroidir, de préférence rapidement, les mèches ainsi traitées à la vapeur, par exemple en envoyant sur ou à travers celles-ci un courant d'air à température ambiante et/ou en aspirant un courant d'air ambiant à travers les mèches.

La production d'un gaz chaud comprenant de la vapeur d'eau peut se faire à l'aide de tout appareil connu en soi et prévu à cet effet. Toutefois, selon la présente invention, on utilise de préférence un appareil tel que celui décrit dans la demande de brevet français FR-A- 2 273 492, ou tout autre appareil équivalent, qui convient en effet dans le cas présent particulièrement bien (traitement ponctuel, régulier et homogène des fibres, sans risque de surchauffe, avec post-traitement de refroidissement intégré).

Les composés amides utilisables selon l'invention ont au moins une chaîne grasse comprenant de préférence de 8 à 40 atomes de carbone.

On peut par exemple utiliser l'amide érucique proposé sous la dénomination CRODAMIDE ER par la société CRODA, ou l'amide béhénique proposé sous la dénomination KEMAMIDE B par la société WITCO.

Les composés amides préférés selon l'invention peuvent répondre à la formule générale (I): 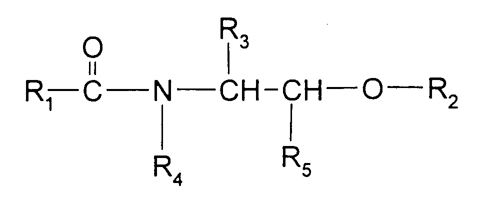 dans laquelle :
- R₁ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ soit un radical R''-(NR-CO)ₙ-R' dans lequel n est égal à 0 ou 1, R désigne hydrogène ou hydroxyéthyle, R' et R'' sont des radicaux hydrocarbonés dont la somme des atomes de carbone est comprise entre 9 et 30, R' étant un radical divalent.
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ;, R₃ peut également désigner un radical α-hydroxyalkyle en C₁₅-C₂₆, le groupement hydroxyle étant éventuellement estérifié par un α-hydroxyacide en C₁₆-C₃₀.
- R₄ désigne un atome d'hydrogène, un radical hydrocarboné en C₁₆-C₂₇, saturé ou insaturé ou un radical -CH₂-CHOH-CH₂-O-R₆ dans lequel R₆ désigne un radical hydrocarboné en C₁₀-C₂₆
- R5 désigne un atome d'hydrogène ou un radical hydrocarboné en C₁-C₄ mono ou polyhydroxylé.

Parmi les composés de formule (I), on préfère les céramides et/ou glycocéramides décrites par DOWNING dans Arch. Dermatol, Vol. 123, 1381-1384, 1987, ou celles décrites dans la demande de brevet français FR-2 673 179.

Les céramides plus particulièrement préférés selon l'invention sont les composés de formule (I) pour lesquels R₁ désigne un alkyle saturé ou insaturé dérivé d'acides gras en C₁₆-C₂₂ ; R₂ désigne un atome d'hydrogène ; et R₃ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

On peut également utiliser les composés de formule (I) pour lesquels R₁ désigne un radical alkyle saturé ou insaturé dérivé d'acides gras ; R₂ désigne un radical galactosyle ou sulfogalactosyle ; et R₃ désigne un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994 et WO94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique.

On peut également utiliser le N-docasanoyl N-méthyl-D-glucamine décrit dans la demande de brevet WO92/05764.

La concentration en amides peut varier entre 0,0001% et 30% en poids environ par rapport au poids total de la composition, et de préférence entre 0,001 et 10% environ.

Les compositions peuvent se présenter sous toute forme habituellement utilisée dans le domaine des compositions capillaires à usage topique, telle que par exemple liquide plus ou moins épaissi ou gélifié, crème, mousse, lotion, gel, pâte, émulsion, aérosol ou toute autre forme appropriée.

Les compositions à base d'amides peuvent ainsi, et d'une manière générale, contenir tous les divers additifs classiques qui sont utilisés dans le domaine de la préparation des compositions capillaires à usage topique et être choisis par exemple parmi des filtres UV, des agents épaississants, des agents de pénétration, des antioxydants, des agents séquestrants, des agents opacifiants, des tampons, des agents tensioactifs choisis parmi les tensioactifs non-ioniques tels que les alkylpolyglycosides, les tensioactifs cationiques, les tensioactifs anioniques et les tensioactifs amphotères, des agents solubilisants, des émollients, des colorants, des parfums et des conservateurs.

Les compositions à base d'amide utilisées dans le cadre de la présente invention, qui sont de préférence destinées à être appliquées sur des cheveux, présentent de préférence un pH compris entre 3 et 11; si nécessaire, ce pH peut être ajusté à la valeur désirée par ajout, selon les cas, soit d'agents basifiants, soit d'agents acidifiants, usuels et connus comme cosmétiquement acceptables.

Des exemples concrets, mais nullement limitatifs, illustrant l'invention vont maintenant être donnés. Aux fins d'une comparaison significative, les mêmes mèches de cheveux de départ (avant traitement) ont été utilisées pour tous les exemples.

### EXEMPLE 1

On a utilisé une composition 1 à base d'amide présentant les caractéristiques suivantes :

| | |
|---|---|
| - N-oleoyl dihydrosphingosine | 0,5 g |
| - Quaternium-27 (REWOQUAT W75 PG) | 2 g |
| - Eau qsp | 100 g |

Cette composition est conditionnée dans un flacon-pompe libérant des doses de 150 µl par pulvérisation.

Le mode opératoire est le suivant : on applique sur une mèche de cheveux décolorés 3 pulvérisations de la composition ci-dessus; puis on traite pendant 3 secondes la mèche au moyen d'un jet de gaz ne contenant pour l'essentiel que de la vapeur d'eau et dont la température est de 85°C; la mèche ainsi traitée est ensuite rapidement refroidie au moyen d'un courant d'air ambiant, enfin la mèche est lavée avec un shampooing standard (0,5 g par mèche pendant 30 secondes) puis rincée 3 fois en effectuant à chaque fois 2 passages dans 200 ml d'eau. La mèche est ensuite séchée à température ambiante.

Pour comparer la quantité d'amide fixé sur les cheveux, on applique la même quantité de composition 1 ci-dessus sur une mèche de cheveux de même qualité que l'on ne traite pas à la vapeur. Le mode opératoire est par ailleurs identique.

Chaque mèche est divisée en deux et on extrait le céramide par extraction au dichlorométhane(2 fois 20 ml pendant 1 heure à température ambiante sous agitation mécanique). Les amides sont ensuite séparés des lipides du cheveu par CCM (Chromatographie sur Couche Mince). L'amide est ensuite dosé par photodensitométrie après carbonisation avec une solution à 3% en poids d'acide sulfurique puis passage à l'étuve à 180°C (on utilise une quantité connue d'amide comme référence).

Les résultats sont rassemblés dans le tableau suivant:

| | Quantité de céramide extraite par g de cheveux |
|---|---|
| Traitement **avec** vapeur | 36 µg ± 1,4 |
| Traitement **sans** vapeur | 16 µg ± 1,4 |

La quantité de céramide fixée sur les cheveux est beaucoup plus importante lorsque les mèches sont traitées à la vapeur d'eau à 85 °C.

### EXEMPLE 2

On a préparé la lotion suivante :

| | |
|---|---|
| - Hydroxycétamide (QUESTAMIDE H de QUEST INT.) | 0,1 g |
| - Ethanol qsp | 100 g |

On a appliqué la composition sur les cheveux de la même façon qu'à l'exemple 1 et on a obtenu des résultats similaires.

### EXEMPLE 3

On a préparé la composition suivante :

| | |
|---|---|
| - N-(2-hydroxyéthyl)-N-(3-cétyloxy-2-hydroxypropyl)amide d'acide cétylique | 0,1 g |
| - Quaternium-27 (REWOQUAT W75 PG) | 0,5 g |
| - Eau qsp | 100 g |

On a appliqué la composition sur les cheveux de la même façon qu'à l'exemple 1 et on a obtenu des résultats similaires.

## Revendications

1. Procédé de traitement des fibres kératiniques humaines, et en particulier des cheveux, caractérisé par le fait qu'il consiste (i) à mettre en contact, pendant une durée n'excédant pas 2 minutes, un gaz contenant de la vapeur d'eau et dont la température est d'au moins 75°C, avec des fibres kératiniques humaines sur lesquelles on a appliqué une composition contenant au moins un composé amide possédant au moins une chaîne grasse à l'exception du diéthanolamide de coprah, (ii) à refroidir les fibres ainsi traitées.

2. Procédé selon la revendication 1, caractérisé par le fait que ledit gaz a une température supérieure ou égale à 85°C.

3. Procédé selon la revendication 2, caractérisé par le fait que ladite température est comprise entre 85°C et 150°C.

4. Procédé selon l'une quelconque des revendications précédentes, caractérisé par le fait que ledit gaz est mis en contact avec la fibre à déformer pendant une durée allant de 0,01 seconde à 2 minutes.

5. Procédé selon la revendication 4, caractérisé par le fait que ladite durée est comprise entre 0,01 seconde et 30 secondes.

6. Procédé selon la revendication 5, caractérisé par le fait que ladite durée est comprise entre 1 seconde et 10 secondes.

7. Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que ledit gaz ne contient que de la vapeur d'eau.

9. Procédé selon l'une quelconque des revendications 1 à 7, caractérisé par le fait que ledit gaz contient de la vapeur d'eau et au moins un autre composé sous forme de gaz ou de vapeur.

10. Procédé selon la revendication 9, caractérisé par le fait que ledit gaz contient de la vapeur d'eau et de l'air.

11. Procédé selon l'une quelconque des revendications précédentes, caractérisé par le fait que les composés amides répondent à la formule générale (I): 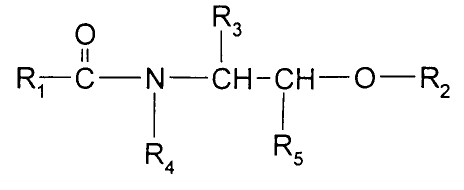 dans laquelle :
- R₁ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R''-(NR-CO)ₙ-R' dans lequel n est égal à 0 ou 1, R désigne hydrogène ou hydroxyéthyle, R' et R'' sont des radicaux hydrocarbonés dont la somme des atomes de carbone est comprise entre 9 et 30, R' étant un radical divalent.
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ;, R₃ peut également désigner un radical α-hydroxyalkyle en C₁₅-C₂₆, le groupement hydroxyle étant éventuellement estérifié par un α-hydroxyacide en C₁₆-C₃₀.
- R₄ désigne un atome d'hydrogène, un radical hydrocarboné en C₁₆-C₂₇, saturé ou insaturé ou un radical -CH₂-CHOH-CH₂-O-R₆ dans lequel R₆ désigne un radical hydrocarboné en C₁₀-C₂₆
- R5 désigne un atome d'hydrogène ou un radical hydrocarboné en C₁-C₄ mono ou polyhydroxylé.

12. Procédé selon l'une quelconque des revendications précédentes, caractérisé en ce que le composé amide est choisi parmi la N-linoléoyldihydrosphingosine, la N-oléoyldihydrosphingosine, la N-palmitoyldihydrosphingosine, la N-stéaroyldihydrosphingosine, la N-béhénoyldihydrosphingosine ou les mélanges de ces composés.

13. Procédé selon l'une quelconque des revendications 1 à 12, caractérisé en ce que le composé amide est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docasanoyl N-méthyl-D-glucamine

14. Procédé selon l'une quelconque des revendications précédentes, caractérisé par le fait que le ou les amides sont présents dans des concentrations allant de 0,0001 à 30% en poids par rapport au poids total de la composition et de préférence de 0,001 à 10 % en poids.

## Claims

1. Process for the treatment of human keratinous fibres, and in particular of hair, characterized in that it consists (i) in placing, for a period not exceeding 2 minutes, a gas which contains steam and the temperature of which is at least 75°C, in contact with human keratinous fibres to which there has been applied a composition containing at least one amide compound which has at least one fatty chain with the exception of coprah diethanolamide and (ii) in cooling the fibres thus treated.

2. Process according to Claim 1, characterized in that the said gas has a temperature higher than or equal to 85°C.

3. Process according to Claim 2, characterized in that the said temperature is between 85°C and 150°C.

4. Process according to any one of the preceding claims, characterized in that the said gas is placed in contact with the fibre to be deformed for a period ranging from 0.01 second to 2 minutes.

5. Process according to Claim 4, characterized in that the said period is between 0.01 second and 30 seconds.

6. Process according to Claim 5, characterized in that the said period is between 1 second and 10 seconds.

7. Process according to any one of the preceding claims, characterized in that the application of the gas is repeated a number of times on the same fibre.

8. Process according to any one of the preceding claims, characterized in that the said gas contains only steam.

9. Process according to any one of Claims 1 to 7, characterized in that the said gas contains steam and at least one other compound in the form of gas or vapour.

10. Process according to Claim 9, characterized in that the said gas contains steam and air.

11. Process according to any one of the preceding claims, characterized in that the amide compounds correspond to the general formula (I): 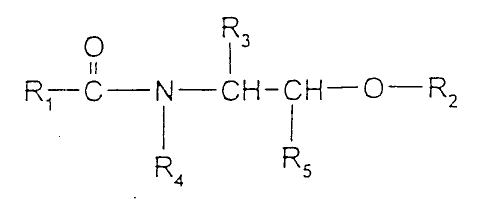 in which:
- R₁ denotes either a C₉-C₃₀, saturated or unsaturated, linear or branched hydrocarbon radical, it being possible for this radical to be substituted by one or more hydroxyl groups optionally esterified by a C₁₆-C₃₀ saturated or unsaturated fatty acid, or a radical R''-(NR-CO)ₙ-R' in which n is equal to 0 or 1, R denotes hydrogen or hydroxyethyl, and R' and R'' are hydrocarbon radicals in which the sum of the carbon atoms is between 9 and 30, R' being a divalent radical,
- R₂ denotes a hydrogen atom or a (glycosyl)ₙ, (galactosyl)ₘ or sulphogalactosyl radical, in which radicals n is an integer varying from 1 to 4 and m is an integer varying from 1 to 8,
- R₃ denotes a hydrogen atom or a saturated or unsaturated C₁₆-C₂₇ hydrocarbon radical, it being possible for this radical to be substituted by one or more C₁-C₁₄ alkyl radicals; R₃ may also denote a C₁₅-C₂₆ α-hydroxyalkyl radical, the hydroxyl group being optionally esterified by a C₁₆-C₃₀ α-hydroxy acid,
- R₄ denotes a hydrogen atom, a saturated or unsaturated C₁₆-C₂₇ hydrocarbon radical or a radical -CH₂-CHOH-CH₂-O-R₆ in which R₆ denotes a C₁₀-C₂₆ hydrocarbon radical,
- R₅ denotes a hydrogen atom or a mono- or polyhydroxylated C₁-C₄ hydrocarbon radical.

12. Process according to any one of the preceding claims, characterized in that the amide compound is chosen from N-linoleoyldihydrosphingosine, N-oleoyldihydrosphingosine, N-palmitoyldihydrosphingosine, N-stearoyldihydrosphingosine, N-behenoyldihydrosphingosine or mixtures of these compounds.

13. Process according to any one of Claims 1 to 12, characterized in that the amide compound is chosen from bis(N-hydroxyethyl-N-cetyl)malonamide, N-(2-hydroxyethyl)-N-(3-cetyloxy-2-hydroxypropyl)cetylamide and N-docosanoyl-N-methyl-D-glucamine.

14. Process according to any one of the preceding claims, characterized in that the amide(s) are present in concentrations ranging from 0.0001 to 30 % by weight relative to the total weight of the composition, and preferably from 0.001 to 10 % by weight.

## Patentansprüche

1. Verfahren zur Behandlung menschlicher Keratinfasern und insbesondere der Haare,
dadurch gekennzeichnet, daß es umfaßt:
(i) Inkontaktbringen während einer Dauer, die nicht mehr als 2 min beträgt, eines Gases, das Wasserdampf enthält und dessen Temperatur mindestens 75 °C beträgt, mit menschlichen Keratinfasern, auf die eine Zusammensetzung aufgebracht wurde, die mindestens eine Amidverbindung enthält, die mindestens eine Fettkette enthält, wobei das Diethanolamid von Kopra ausgenommen ist, und
(ii) Abkühlen der so behandelten Fasern.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß die Temperatur 85 bis 150 °C beträgt.

4. Verfahren nach einem der vorhergehenden Ansprüche, dadurch gekennzeichnet, daß das Gas mit der zu verformenden Faser während einer Dauer von 0,01 s bis 2 min in Kontakt gebracht wird.

5. Verfahren nach Anspruch 4, dadurch gekennzeichnet, daß die Dauer 0,01 bis 30 s beträgt.

6. Verfahren nach Anspruch 5, dadurch gekennzeichnet, daß die Dauer 1 bis 10 s beträgt.

7. Verfahren nach einem der vorhergehenden Ansprüche, dadurch gekennzeichnet, daß man das Gas mehrmals auf eine Faser einwirken läßt.

8. Verfahren nach einem der vorhergehenden Ansprüche, dadurch gekennzeichnet, daß das Gas ausschließlich Wasserdampf enthält.

9. Verfahren nach einem der Ansprüche 1 bis 7, dadurch gekennzeichnet, daß das Gas den Wasserdampf und mindestens eine weitere gasförmige oder dampfförmige Verbindung enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, daß die Amidverbindungen die allgemeine Formel (I) 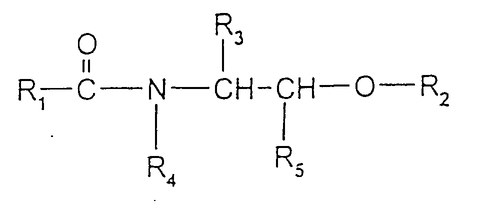 aufweisen, worin bedeuten:
- R₁ einen gesättigten oder ungesättigten, geradkettigen oder verzweigten Kohlenwasserstoffrest mit 9 bis 30 Kohlenstoffatomen, wobei dieser Rest ggf. mit einer oder mehreren Hydroxygruppen substituiert ist, die ggf. mit einer gesättigten oder ungesättigten Fettsäure mit 16 bis 30 Kohlenstoffatomen verestert sind, oder einen Rest R''-(NR-CO)ₙ-R', in dem n gleich 0 oder 1 ist, R Wasserstoff oder Hydroxyethyl bedeutet und R' und R'' Kohlenwasserstoffreste sind, deren Summe der Kohlenstoffatome 9 bis 30 beträgt, wobei R' ein zweiwertiger Rest ist,
- R₂ Wasserstoff oder einen (Glykosyl)ₙ-, (Galactosyl)ₘ- oder Sulfogalactosylrest, worin n eine ganze Zahl im Bereich von 1 bis 4 und n eine ganze Zahl im Bereich von 1 bis 8 ist,
- R₃ Wasserstoff, einen gesättigten oder ungesättigten Kohlenwasserstoffrest mit 16 bis 27 Kohlenstoffatomen, wobei dieser Rest ggf. mit einem oder mehreren Alkylgruppen mit 1 bis 14 Kohlenstoffatomen substituiert ist, oder einen α-Hydroxyalkylrest mit 15 bis 26 Kohlenstoffatomen, wobei die Hydroxygruppe ggf. mit einer α-Hydroxysäure mit 16 bis 30 Kohlenstoffatomen verestert ist,
- R₄ Wasserstoff, einen gesättigten oder ungesättigten Kohlenwasserstoffrest mit 16 bis 27 Kohlenstoffatomen oder eine Gruppe -CH₂-CHOH-CH₂-O-R₆, in der R₆ einen Kohlenwasserstoffrest mit 10 bis 26 Kohlenstoffatomen darstellt,
- R₅ Wasserstoff oder einen mono- oder polyhydroxylierten Kohlenwasserstoffrest mit 1 bis 4 Kohlenstoffatomen.

12. Verfahren nach einem der vorhergehenden Ansprüche, dadurch gekennzeichnet, daß die Amidverbindung ausgewählt wird unter *N*-Linoleoyldihydrosphingosin, *N*-Oleoyldihydrosphingosin, *N*-Palmitoyldihydrosphingosin, *N*-Stearoyldihydrosphingosin, *N*-Behenoyldihydrosphingosin und den Gemischen dieser Verbindungen

13. Verfahren nach einem der Ansprüche 1 bis 12, dadurch gekennzeichnet, daß die Amidverbindung ausgewählt wird unter Bis(*N*-hydroxyethyl-*N*-cetyl)malonamid, dem *N*-(2-Hydroxyethyl)-*N*-(3-cetyloxy-2-hydroxypropyl)-amid der Cetylsäure und *N*-Docasanoyl-*N*-methyl-D-glucamin.

14. Verfahren nach einem der vorhergehenden Ansprüche, dadurch gekennzeichnet, daß das oder die Amide in einer Konzentration von 0,0001 bis 30 Gew.-%, bezogen auf das Gesamtgewicht der Zusammensetzung, und vorzugsweise 0,001 bis 10 Gew.-% enthalten sind.
